# EUROPEAN PATENT APPLICATION

(11) **EP 4 289 938 A1**
(43) Date of publication of application: **13.12.2023**
(21) Application number: 22750007.1
(22) Date of filing: 28.01.2022
(51) Int. Cl.: C12N 5/0775, C07K 14/705, A61K 47/69

(54) **CELL-DERIVED VESICLES WITH INCREASED CELLULAR UPTAKE CAPACITY AND METHOD FOR PRODUCING SAME**

(30) Priority: 02.02.2021 KR 20210014500
(71) Applicant: MDimune Inc., Seoul 04790 (KR)
(72) Inventor: BAE, Shin Gyu, Seoul 04790 (KR); OH, Seung Wook, Seoul 04790 (KR); PARK, Jung Eun, Seoul 04790 (KR); PARK, Sung Soo, Seoul 04790 (KR); KWON, Ye Rim, Seoul 04790 (KR); LAU, Hui Chong, Seoul 04791 (KR); PARK, Jin Hee, Seoul 04790 (KR)
(74) Representative: Boult Wade Tennant LLP
(86) International application number: PCT/KR2022/001673
(87) International publication number: WO 2022/169258

(57) **Abstract**

The present invention relates to cell-derived vesicles with increased cellular uptake capacity and a method for producing same. The cell-derived vesicles of the present invention are produced by migrating cells to micropores, and exhibit the characteristic of expressing a protein marker that is different from that of exosomes naturally secreted by cells. The cell-derived vesicles with increased cellular uptake capacity, according to the present invention, have remarkably superior cellular uptake capacity as compared to natural exosomes secreted by cells, and thus can be effectively used to deliver various active substances, such as drugs and marker substances, into target cells.

## Description

### [Technical Field]

The present invention relates to cell-derived vesicles with increased cellular uptake capacity and a method for producing the same. The cell-derived vesicles of the present invention are produced by migrating cells into micropores, and exhibit the characteristic of expressing a membrane surface protein marker that is different from that of exosomes naturally secreted by cells.

### [Background Art]

Methods of directly introducing a target protein into cells have been frequently studied. For example, a recombinant protein of a protein transduction domain (PTD), which is a peptide that passes through the cell membrane, and a target protein may be prepared so that the recombinant protein passes through the cell membrane to be introduced into the cytoplasm. Human immunodeficiency virus type-1 transactivating regulatory protein (HIV-1 TAT), HSV VP22, Antp, dfTAT, Hph-1, and the like are widely known protein transduction domains. However, the method using the PTD has problems in that the refolding of the protein is not properly performed in a process of producing and isolating a fusion protein, in which the PTD and the target protein are bound, in the form of a recombinant protein, and as a result, the activity of the produced protein is low, the protein is delivered non-specifically, and the yield is low.

A method of delivering a target protein into cells by binding a target protein to the nanoparticle using various nanoparticles such as Gold nano particle (NP), Liposome NP, Magnetic NP, Polymeric NP, etc. is also widely used. The target protein bound to the nanoparticles may pass through the cell membrane to be introduced to the cytoplasm by endocytosis. However, most of conjugates of nanoparticles and target proteins are degraded in intracellular lysosomes, have a disadvantage that the activity of the protein may be lost through this degradation process, and have a problem in that since it is difficult to separately isolate the target protein and the nanoparticles, toxicity may be caused by the nanoparticles.

Exosomes refer to small membrane-structured vesicles with sizes of 50 to 200 nm that are secreted out of cells while capturing proteins, DNA, RNA, etc., for intercellular signaling. The exosomes are not directly separated from the plasma membrane, but released and secreted out of the cells by originating from a specific intracellular compartment called a multivesicular body (MVB). The exosomes are variously produced and secreted by various types of immune cells including B-lymphocytes, T-lymphocytes, dendritic cells, megakaryocytes, and macrophages, stem cells, tumor cells, etc. The exosomes include various proteins, DNA, RNA, etc. in cells, and these substances are re-introduced into other cells by fusion with the cell membrane or endocytosis and serve as a communicator between cells. Therefore, since exosomes containing the target protein therein are expected to be used for the treatment of various diseases *in vivo,* an intracellular delivery method using exosomes has recently been studied using the same.

In the intracellular delivery method using exosomes, various studies have been conducted on methods for efficiently producing exosomes containing a target protein and methods for increasing production of exosomes by releasing the exosomes out of cells, but new exosomes with excellent cellular uptake capacity have not yet been widely reported.

### [Disclosure]

### [Technical Problem]

Accordingly, the present inventors have studied new cell-derived vesicles that may be absorbed into cells better to effectively deliver a target substance into cells, confirmed that cell-derived vesicles produced by migrating cells into micropores exhibited a different expression pattern of a membrane surface protein marker as compared to that of exosomes naturally secreted by cells, and better cellular uptake capacity, and then completed the present invention.

Therefore, an object of the present invention is to provide cell-derived vesicles with increased cellular uptake capacity and a method for producing the same.

### [Technical Solution]

An aspect of the present invention provides a method for producing cell-derived vesicles (CDVs) with increased cellular uptake capacity including producing cell-derived vesicles by migrating a sample containing cells into micropores.

Another aspect of the present invention provides cell-derived vesicles with increased cellular uptake capacity characterized by increasing the expression of at least one membrane surface protein marker selected from the group consisting of CD63 and LAMP1 as compared to cell-secreted exosomes.

Yet another aspect of the present invention provides cell-derived vesicles with increased cellular uptake capacity produced by a method including producing cell-derived vesicles by migrating a sample containing cells into micropores.

Still another aspect of the present invention provides a composition for use in delivering an active ingredient including the cell-derived vesicles with increased cellular uptake capacity.

Still yet another aspect of the present invention provides a method for delivering an active ingredient into a subject *in vitro* or *ex vivo* including treating the cell-derived vesicles with increased cellular uptake capacity including the active ingredient to a subject.

Still yet another aspect of the present invention provides a method for delivering an active ingredient into a subject including treating the cell-derived vesicles with increased cellular uptake capacity to a subject.

### [Advantageous Effects]

According to the present invention, the cell-derived vesicles with increased cellular uptake capacity have remarkably superior cellular uptake capacity as compared to natural exosomes secreted by cells, and thus can be effectively used to deliver various active substances, such as drugs and marker substances, into target cells.

### [Description of Drawings]

FIG. 1 is a diagram illustrating results of confirming uptake capacity of UCMSC-CDVs and UCMSC-secreted exosomes into BT549 over time through DiR fluorescence labeling. As control groups, a sample added with DiR to PBS, unlabeled CDVs, and unlabeled exosomes were used.
FIG. 2 is a diagram illustrating results of confirming cellular uptake of UCMSC-CDVs and UCMSC-secreted exosomes through a confocal microscope.
FIG. 3 is a diagram illustrating results of confirming membrane surface protein markers of CDVs and exosomes derived from UCMSC, HEK293 (adherent), and HEK293 (suspension) cells through protein immunoblot analysis.
FIG. 4 is a diagram illustrating the expression of membrane surface protein markers of CDVs and exosomes derived from UCMSC, HEK293 (adherent), and HEK293 (suspension) cells quantified through an Image J program (mean ± SD, * p < 0.05; ** p < 0.01; *** p < 0.001, unpaired two-tailed T test).
FIG. 5 is a diagram illustrating results of confirming changes in expression of membrane surface protein markers by treating Protease K to CDVs and exosomes derived from UCMSC and HEK293 (suspension) cells (mean ± SD, * p < 0.05; ** p < 0.01; *** p < 0.001, unpaired two-tailed T test).
FIG. 6 is a diagram illustrating results of confirming changes in cellular uptake capacity by treating Protease K to CDVs and exosomes derived from UCMSC and HEK293 (suspension) cells (mean ± SD, ** p < 0.01; *** p < 0.001, unpaired two-tailed T test).
FIG. 7 is a diagram illustrating results of confirming changes in cellular uptake capacity by performing concentration-specific blocking with each membrane surface protein-specific antibody on UCMSC-derived CDVs and exosomes (mean ± SD, *p < 0.05; ** p < 0.01; *** p < 0.001, unpaired two-tailed T test).
FIG. 8 is a diagram illustrating results of confirming changes in cellular uptake capacity by performing concentration-specific blocking with each membrane surface protein marker-specific antibody on HEK293 (suspension) cells-derived CDVs and exosomes (mean ± SD, *p < 0.05; ** p < 0.01; *** p < 0.001, unpaired two-tailed T test).

### [Best Mode of the Invention]

The present invention provides cell-derived vesicles (CDVs) with increased cellular uptake capacity, a method for producing the same, and a method for delivering an active substance into cells using the same.

In the present invention, the "cell-derived vesicles" may be vesicles with increased cellular uptake capacity, and may be produced by migrating a sample containing cells into micropores. Accordingly, the present invention relates to a method for producing cell-derived vesicles (CDVs) with increased cellular uptake capacity including producing cell-derived vesicles by migrating a sample containing cells into micropores.

In the present invention, the "cell-derived vesicles" may be increased in cellular uptake capacity as compared with originated cells or exosomes naturally secreted by originated cells.

The exosomes naturally secreted by the cells refer to exosomes secreted by cells without separate stimulation or treatment, and may be exosomes isolated from cells by a centrifugation method as a method known in the art. In the present invention, the exosomes naturally secreted by cells as described above are referred to as 'cell-secreted exosomes.'

The cell-derived vesicles (CDVs) of the present invention produced by migrating cells into micropores may be increased in expression of at least one membrane surface protein marker selected from the group consisting of CD29, flotillin-1, CD63, LAMP1, Calnexin and GM130; or decreased in expression of at least one membrane surface protein marker selected from the group consisting of CD81 and CD9 as compared with the cell-secreted exosomes described above. Preferably, the CDVs may be increased in expression of at least two, three, four, and five membrane surface protein markers selected from the group consisting of CD29, flotillin-1, CD63, LAMP1, Calnexin and GM130 or all markers of CD29, flotillin-1, CD63, LAMP1, Calnexin and GM130 or decreased in expression of both CD81 and CD9 as compared with the cell-secreted exosomes. In the present invention, the increase compared to exosomes means a significant increase in expression, such as at least 2-fold to at most 59-fold increase.

In the present invention, the "cell-derived vesicle (CDV)" is a vesicle artificially prepared in a cell and has a form of a double phospholipid membrane. The cell-derived vesicles of the present invention are particularly increased in expression of membrane surface protein markers CD63 and LAMP1 related to cellular uptake capacity compared to cell-secreted exosomes, thereby increasing cellular uptake capacity.

In order to produce the cell-derived vesicles of the present invention, nucleated cells or transformed cells thereof may be used, but any cell capable of producing vesicles may be used without limitation, preferably stem cells, undifferentiated cells, immune cells, somatic cells, induced pluripotent stem cells or germ cells. In addition, the cells of the present invention may be at least one cell selected from the group consisting of umbilical cord-derived mesenchymal stem cells, Wharton's jelly-derived mesenchymal stem cells, placenta-derived mesenchymal stem cells, tonsil-derived mesenchymal stem cells, adipose-derived mesenchymal stem cells, umbilical cord blood-derived mesenchymal stem cells, bone marrow-derived mesenchymal stem cells, and embryonic kidney cells. In the present invention, as a specific embodiment, human embryonic kidney suspension and adherent cells (HEK293), and umbilical cord-derived mesenchymal stem cells were used.

The vesicles of the present invention may be produced by using a method selected from the group consisting of extrusion, sonication, lysis, homogenization, freeze-thawing, electroporation, chemical treatment, mechanical degradation, and treatment with a physical stimulus applied externally to the cells for a suspension containing cells, but is not limited thereto. In the present invention, the vesicles were produced by using a cell extruder of an extrusion method using micropores, which migrates and extrudes the suspension sample containing the cells sequentially from a filter with a large pore size to a filter with a small pore size by applying pressure.

More specifically, in the present invention, the "producing of the cell-derived vesicles by migrating the sample into the micropores" may be a step of migrating the sample by applying a pressure to a filter having a filterable membrane structure with a pore size of 1 to 10 µm, preferably a step of migrating the sample sequentially from a filter having a large pore size to a filter having a small pore size.

Further, the present invention provides cell-derived vesicles with increased cellular uptake capacity characterized by increasing the expression of at least one membrane surface protein marker selected from the group consisting of CD63 and LAMP1 as compared to cell-secreted exosomes.

In the present invention, it was confirmed that the cell-derived vesicles of the present invention had significantly increased cellular uptake capacity as compared to existing cell-secreted exosomes, and it was confirmed that the excellent cellular uptake capacity was caused by membrane surface proteins exhibiting a significant expression difference in the cell-derived vesicles. In particular, in the present invention, it was confirmed that the membrane surface protein markers were related to increased cellular uptake capacity by confirming changes in cellular uptake capacity through increased expression of CD63 and LAMP1 and specific blocking thereof. Accordingly, the present invention provides cell-derived vesicles with increased cellular uptake capacity characterized by increasing the expression of at least one membrane surface protein marker selected from the group consisting of CD63 and LAMP1 as compared to cell-secreted exosomes.

In addition to the membrane surface marker, the cell-derived vesicles may be increased in expression of one, two, three or four membrane surface protein markers selected from the group consisting of CD29, flotillin-1, Calnexin and GM130; or decreased in expression of at least one or both selected from the group consisting of CD81 and CD9 as compared to cell-secreted exosomes.

In the present invention, the cell-derived vesicles with increased cellular uptake capacity may be produced by using all methods known to those skilled in the art without limitations, capable of producing vehicles with increased expression of at least one membrane surface protein marker selected from the group consisting of CD63 and LAMP1 as compared to cell-secreted exosomes. However, preferably, the cell-derived vesicles may be produced by a method including producing cell-derived vesicles by migrating the sample containing cells into micropores.

Further, the present invention provides cell-derived vesicles with increased cellular uptake capacity produced by a method including producing cell-derived vesicles by migrating a sample containing cells into micropores.

The cell-derived vesicles with increased cellular uptake capacity may be characterized by having increased expression of at least one membrane surface protein marker selected from the group consisting of CD63 and LAMP1 as compared to cell-secreted exosomes. Additionally, the cell-derived vesicles may be increased in expression of one, two, three or four membrane surface protein markers selected from the group consisting of CD29, flotillin-1, Calnexin and GM130; or decreased in expression of at least one or both selected from the group consisting of CD81 and CD9 as compared to cell-secreted exosomes.

In the present invention, the "producing of the cell-derived vesicles by migrating the sample into the micropores" may be a step of migrating the sample by applying a pressure to a filter having a filterable membrane structure with a pore size of 1 to 10 µm, preferably a step of migrating the sample sequentially from a filter having a large pore size to a filter having a small pore size.

Further, the present invention provides a composition for use in delivering an active ingredient including the cell-derived vesicles with increased cellular uptake capacity.

By using the composition for delivering the active ingredient, the active ingredient may be more effectively delivered to cells, tissues, organs, or mammals including humans.

Further, the present invention provides a method for delivering an active ingredient into a subject *in vitro* or *ex vivo* including treating the cell-derived vesicles with increased cellular uptake capacity including the active ingredient according to the present invention to a subject. Further, the present invention provides a method for delivering an active ingredient into a subject including treating the cell-derived vesicles with increased cellular uptake capacity to a subject.

In the present invention, the subject treated with the cell-derived vesicles with increased cellular uptake capacity may be cells, tissues, organs, or mammals including humans, and may be cells, organs, or tissues when the delivery method is performed *in vitro* or *ex vivo.*

Since the cell-derived vesicles included in the composition for delivering the active ingredient or used in the method may be absorbed into cells with higher efficiency than existing cell-secreted exosomes, the active ingredient may be delivered more effectively. At this time, the cell-derived vesicles may be in a state where the active ingredient to be delivered is included or supported inside or outside the vesicles, or the active ingredient is bound.

The active ingredient that may be delivered to the cells through the cell-derived vesicles of the present invention may include any substance capable of exhibiting its desired effect by being delivered into cells without limitation. However, the active ingredient may be preferably at least one selected from the group consisting of an antifungal agent, an antibacterial agent, an antimicrobial agent, an antioxidant, a cooling agent, a soothing agent, a wound healing agent, an anti-inflammatory agent, an anti-aging agent, an anti-wrinkle agent, a skin whitening agent, an anticancer agent, an angiogenesis inhibitor, peptides, proteins, toxins, nucleic acids, beads, and microparticle and nanoparticle proteins and compounds.

In this case, the composition for delivering the active ingredient into the cells may be a pharmaceutical composition, and may also include a carrier, a diluent, an excipient, or a combination of two or more thereof, which is commonly used in biological preparations. The pharmaceutically acceptable carrier is not particularly limited as long as the pharmaceutically acceptable carrier is suitable for delivering the composition *in vivo,* and examples thereof may include a compound, saline, sterile water, Ringer's solution, buffered saline, dextrose solution, maltodextrin solution, glycerol, ethanol or a mixture of one or more ingredients thereof. At this time, other conventional additives such as an antioxidant, a buffer, and a bacteriostatic agent may be added if necessary. In the case of formulating the composition, the formulation may be prepared by using a diluent or an excipient, such as a filler, an extender, a binder, a wetting agent, a disintegrating agent, and a surfactant which are generally used.

The composition of the present invention may be formulated as an oral preparation or parenteral preparation. Solid formulations for oral administration include tablets, pills, powders, granules, capsules, troches, etc., and these solid formulations may be prepared by mixing at least one excipient, for example, starch, calcium carbonate, sucrose, lactose, gelatin, and the like with at least one composition. In addition, lubricants such as magnesium stearate and talc may also be added. Meanwhile, liquid formulations correspond to suspensions, internal solutions, emulsions, syrups, and the like, which may include excipients such as a wetting agent, a sweetener, an aromatic, and a preservative.

Formulations for parenteral administration may include injections such as a sterile aqueous solution, a non-aqueous solution, a suspension, an emulsion, and the like.

As the non-aqueous solution and the suspension, propylene glycol, polyethylene glycol, vegetable oil such as olive oil, injectable ester such as ethyl oleate, and the like may be used.

The composition of the present invention may be administered orally or parenterally, depending on a desired method. The parenteral administration may be selected from various injection methods such as external skin or intraperitoneal injection, intrarectal injection, subcutaneous injection, intravenous injection, intramuscular injection, intrathoracic injection, intranasal administration, intravitreal injection, intrathecal injection or intracerebroventricular injection, but is not limited thereto.

The composition according to the present invention is administered in a pharmaceutically effective dose. The pharmaceutically effective dose may vary depending on the type and severity of a disease, the activity of a drug, the sensitivity to a drug, an administration time, an administration route and an excretion rate, a treatment period, concurrently used drugs, etc. The composition of the present invention may be administered alone or in combination with other therapeutic agents. When administered in combination, administration may be sequential or simultaneous.

Duplicated contents are omitted in consideration of the complexity of the present specification, and terms not defined otherwise in the present specification have the meanings commonly used in the art to which the present invention pertains.

### [Modes for the Invention]

### Example 1. Preparation of samples

Cell-derived vesicles (CDVs) and exosomes isolated in a cell-secreted state were obtained, respectively, from umbilical cord-derived mesenchymal stem cells (UCMSCs) and human embryonic kidney suspension cells (HEK293 suspension cells) and adherent cells (HEK293 adherent cells) to be prepared as samples.

Each sample was specifically prepared as follows.

### 1.1 Umbilical cord-derived mesenchymal stem cells (UCMSCs)

Umbilical cord-derived mesenchymal stem cells (UCMSCs) were produced under the Good Manufacturing Practice (GMP) facility of Paracelsus Medical Private University (PMU). The UCMSCs were inoculated in a double-layered CellSTACKs^{®} (Corning, NY, USA) including a DMEM low-glucose medium (Gibco BRL, MA, USA) added with 4% human platelet lysate (hPL; Helios, AventaCell Biomedica, GA, USA) and 1% penicillin-streptomycin (Gibco BRL, MA, USA). The cells were cultured in an environment of 37°C and 5% carbon dioxide, and cell growth was monitored daily. After twice subculture, the cells were obtained at a concentration of 2.5 × 10⁵ cells/mL and resuspended in PBS (Gibco BRL, MA, US). The cell suspension was extruded sequentially through filters having pore sizes of 10, 3, and 0.4 µm (Whatman Inc, NJ, USA) to obtain UCMSC-derived cell-derived vesicles (hereinafter, CDVs). Thereafter, the CDVs were purified by a tangential flow filtration (TFF, Repligen CA, US) device by passing through a 0.45 µm pore filter (Sartorious, Gottingen, Germany) through a filter using MidiKros 750 kDa molecular weight cutoff (MWCO) hollow fibers. The CDVs were further purified by size exclusion chromatography using qEV10 (Izon, MA, US) for Western blot.

In order to obtain UCMSC-derived exosomes, first, UCMSCs were cultured using the same cell culture method and culture medium composition as described above. After twice subculture, when the cell confluency was about 80%, the medium was replaced with a DMEM low-glucose medium added with 4% of exosome-filtered human platelet lysate (hPL) and 1% of penicillin-streptomycin. After 24 hours of culture, the obtained culture medium was sequentially centrifuged at 300 X g for 10 minutes, 2,000 X g for 10 minutes, and 10,000 X g for 30 minutes to remove cell debris and dead cells, and then ultracentrifuged at 120,000 X g for 2 hours (Beckman Coulter, CA, US). A supernatant was removed and the cells were resuspended in PBS and then ultracentrifuged again at 120,000 X g for 2 hours to obtain exosomes. The purified UCMSC-derived cell-secreted exosomes were used in subsequent experiments.

### 1.2 Human embryonic kidney suspension cells (HEK293 suspension cells) and adherent cells (HEK293 adherent cells)

Human embryonic kidney suspension cells (HEK293 suspension cells, hereinafter HEK293 (suspension)) were inoculated in a 250 mL flask containing a FreeStyle F17 medium added with 4 mM Glutamax (Glutamax, Gibco BRL, USA) and 0.2% Pluronic F68 (Gibco, BRL USA). The cells were cultured at 120 rpm in a 37°C, 8% carbon dioxide environment, and twice subculture was performed. The cells were harvested and then resuspended in PBS at a concentration of 5 × 10⁵ cells/mL. HEK293 (suspension)-CDVs were obtained by extracting CDVs from HEK293 (suspension) cells in the same manner as the method of obtaining CDVs from UCMSC cells, and purified using a TFF device to remove impurities. To perform a protein immunoblot experiment, CDVs were further isolated and purified by size exclusion chromatography using qEV10 (Izon, MA, US).

In order to obtain HEK293 (suspension)-derived exosomes, first, HEK293 (suspension) was cultured using the same cell culture method and culture medium composition as described above. After twice subculture, the culture medium was obtained and ultracentrifuged at 10,000 g for 30 minutes, and the supernatant was taken and purified in the same manner as when HEK293 (suspension)-CDV was purified.

Human embryonic kidney adherent cells (HEK293 adherent cells, hereinafter HEK293 (adherent)) were inoculated in a MEM alpha medium (Gibco, BRL, MA, US) added with 10% FBS and 1% penicillin-streptomycin in a T175 flask (Thermo Scientific, MA, USA). The cells were cultured under 37°C, 5% carbon dioxide conditions, and after twice subculture, the cells were harvested and resuspended in PBS at a concentration of 5 × 10⁵ cells/mL. Thereafter, HEK293 (adherent)-CDVs and HEK293 (adherent)-exosomes were produced from the HEK293 (adherent) cells in the same manner as in the HEK293 (suspension) cells.

CDVs and exosomes isolated from respective cells were used in subsequent experiments.

### Example 2. Comparison of cellular uptake capacity of CDVs and cell-secreted exosomes

An experiment was performed to determine whether there was a difference in uptake capacity of UCMSC-derived CDVs and exosomes obtained in Example 1 into cells. As target cells, a triple negative breast cancer cell line (BT549) was used. 1 × 10¹¹ particles of CDVs and cell-secreted exosomes prepared in Example 1 were treated with 5 µM DiR (Thermo Scientific, MA, US), and cultured at 37°C for 30 minutes to label the membrane. DiR not used for labeling was removed by size exclusion chromatography (PD-10, GE, IL, US) according to the manufacturer's instructions. As a control group, a DiR control was treated with 5 µM DiR in PBS and then subjected to the same process as the labeling method for the CDVs and exosomes. Control groups of CDVs and exosomes were prepared in the same manner except that DiR was replaced with PBS.

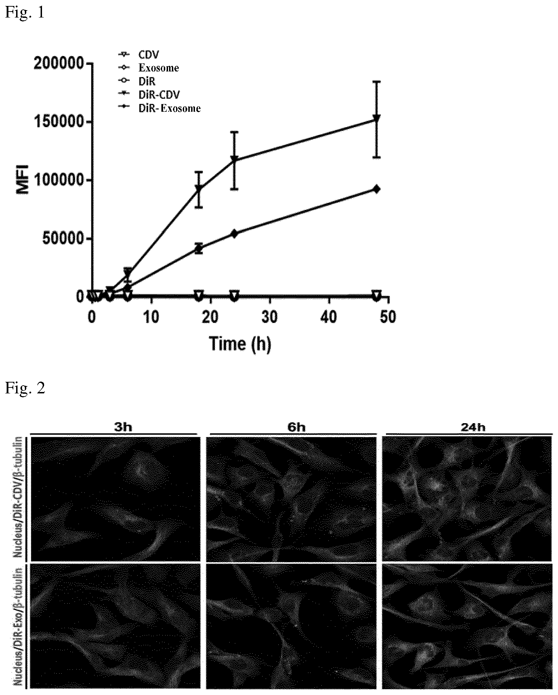

The triple-negative breast cancer cell line was spread on a 24-well plate (for flow cytometer) or a glass coverslip (for microscopic analysis) at a cell density of 4 × 10⁴, and cultured for 17 hours or more for cell adhesion. The cultured triple-negative breast cancer cell line was treated with DiR-labeled CDVs (DiR-CDV) and exosomes (DiR-exosomes), and a control group to be 1 × 10⁵ particles/cell and cultured at 37°C for 1, 3, 6, 18, 24, and 48 hours, respectively. After measuring the fluorescence intensities of the control groups (DiR control, CDVs, exosomes), DiR-CDV and DiR-exosomes over time using a flow cytometer, the cellular uptake rates were quantified and shown in a graph. The results of comparing the cellular uptake rates of CDVs and exosomes were shown in FIG. 1.

As shown in FIG. 1, it was confirmed that fluorescence values were not detected regardless of time in control groups, unlabeled UCMSC-CDVs (CDV), UCMSC-exosomes (exosomes), and DiR control (DiR), whereas the fluorescent intensities of DiR-CDVs and DiR-exosomes were increased over time. This indicated that both DiR-CDVs and DiR-exosomes were efficiently absorbed into cells of BT549 over time, and CDVs showed about twice larger cellular uptake than exosomes at all observed times.

In addition, in order to directly observe and compare the cellular uptake of CDVs and exosomes, the cellular uptake rates of CDVs and exosomes over time were confirmed by immunofluorescence staining images using a confocal microscope. BT549 cells treated with DiR-labeled UCMSC-CDVs (DiR-CDVs) (red) or DiR-labeled UCMSC-exosomes (DiR-Exo) (red) for 3, 6, and 24 hours were stained with Alexa Fluor-488 β-tubulin (cytoplasm; green) and Hoechst (nucleus; blue), and after each reaction time, the results of observing the BT549 cells using a confocal microscope were shown in FIG. 2.

As shown in FIG. 2, CDVs exhibited greater cellular uptake than cell-secreted exosomes, which was the result matched with the FACS results confirmed above. The high cellular uptake capacity of CDVs as described above was a result showing that CDVs were more suitable for an intracellular drug delivery system than cell-secreted exosomes.

### Example 3. Surface protein analysis of CDVs and cell-secreted exosomes

Through Example 2, it was confirmed that CDVs had excellent cellular uptake capacity as compared to cell-secreted exosomes, and an experiment was conducted to confirm whether a difference in cellular uptake capacity was due to a difference in membrane surface protein. First, protein immunoblot analysis was performed to identify surface proteins present on each CDV and cell-secreted exosome.

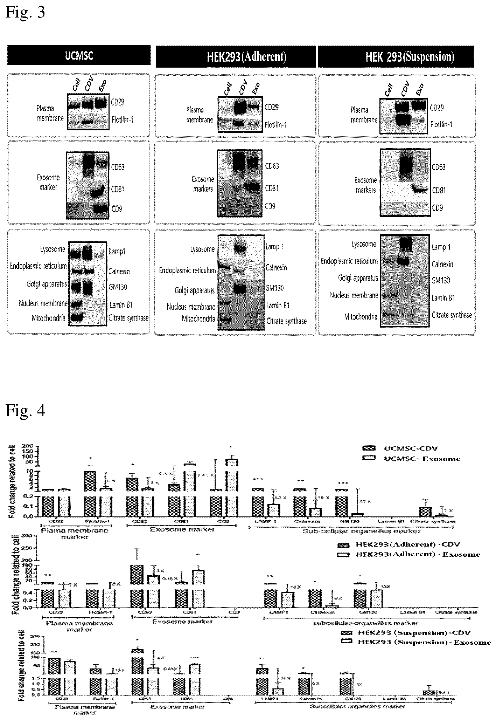

Exosome markers CD9, CD29, CD63, and CD81 and organelle markers Flotilin-1, LAMP1, Calnexin, GM130, Lamin B1, and Citrate synthase included in UCMSC, HEK293 (adherent) and HEK293 (suspension) cells and CDVs and exosomes produced from the cells were separated by a BoltTM 4-12% Bis-Tris Plus gel (Invitrogen, MA, US) and the proteins were migrated to a PVDF membrane (Life Technologies, MA, US). The membrane reacted with primary antibodies anti-CD63 (Life Technologies, MA, US), anti-CD81 (Abcam, MA, US), anti-CD9 (Life Technologies, MA, US), anti-LAMP1, anti-calnexin (Abcam, MA, US), anti-CD29 (Abcam, US), anti-GM130 (Abcam, MA, US), anti-citrate-synthase (Abcam, MA, US), anti-flotillin-1 (Cell signaling, MA, US), and anti-lamin B1 (Abcam, MA, US) at a dilution ratio of 1 : 2500 for 17 hours or more. In the next step, the membrane was rinsed with 1X TBST (Biosensang, Seoul, Korea), and secondary antibodies were added at a dilution ratio of 1 : 2500 and reacted for 1 hour. After rinsing again with 1X TBST, each protein was identified using a Gel-Doc (Biorad, CA, US) system using an Amersham ECL Prime western blotting detection reagent (GE, IL, US). The intensities of the protein bands were quantified by the intensity of the protein band obtained from the cells using an Image J program, and then the measured values were shown. The protein immunoblot analysis results and the quantification results through the Image J program were shown in FIGS. 3 and 4.

As can be seen in FIGS. 3 and 4, CD63 and flotillin-1 were expressed at least three-fold higher in CDVs than in exosomes, and organelle markers such as Calnexin, LAMP1 and GM130 were expressed at least 6-fold higher in CDVs than in exosomes, whereas CD9 and CD81 were expressed at least 7-fold lower in CDVs than in exosomes. In addition, Lamin B1 and citrate synthase were expressed at low levels in CDVs and exosomes compared to other protein markers.

Among the markers, CD29 was expressed 7-fold higher in HEK293 (adherent)-CDVs than in HEK293 (adherent)-exosomes, and CD9 was expressed approximately 100-fold lower in UCMSC-CDVs than in UCMSC-exosomes.

Through the above results, it was confirmed that the CDVs of the present invention exhibited significantly different marker expression patterns as compared to cell-secreted exosomes isolated by a centrifugation method, and also exhibited different marker expression patterns from the originated cells.

### Example 4. Comparison of cellular uptake rate according to membrane surface proteinase treatment

Through Examples 2 and 3, it was confirmed that CDVs showed a higher cellular uptake rate than cell-secreted exosomes and exhibited a significant difference in the expression of membrane surface protein markers. It was confirmed whether the significant difference in expression of these membrane surface protein markers was associated with the difference in cellular uptake rate. Specifically, CDVs and cell-secreted exosomes derived from UCMSC and HEK293 (suspension) cells were treated with proteinase K to degrade CDV and exosome surface protein markers. Then, changes in the cellular uptake rate of CDVs and exosomes according to proteinase K treatment were compared.

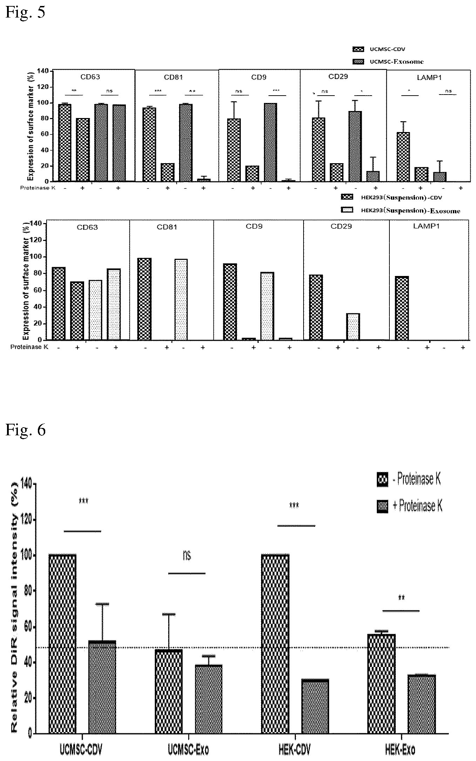

First, an experiment was performed to confirm whether CDV or exosome surface protein markers were well degraded according to proteinase treatment. More specifically, CDVs and exosomes were produced from UCMSC and HEK293 (suspension) cells by the method of Example 1. Then, the protein concentrations of CDVs and exosomes were measured by bicinchoninic acid (BCA) assay. Thereafter, CDVs and exosomes were treated with proteinase K (Thermo Scientific) at a concentration of 2 µg proteinase K/µg protein, vortexed once every 15 minutes, and cultured at 37°C for 1 hour. Thereafter, the samples were heated at 90°C for 5 minutes to inhibit the activity of proteinase. The degrees of degradation of membrane proteins were analyzed by a flow cytometer (FACS, Sony Japan) using 5 µm aldehyde beads (Thermo Scientific, MA, US). Changes in expression of CD63, CD81, CD9, CD29, and LAMP1, which were surface marker proteins, were confirmed in CDVs and exosomes derived from UCMSC and HEK293 (suspension) cells, and the results were shown in FIG. 5.

As shown in FIG. 5, it was confirmed that the expression levels of protein markers CD81, CD9, CD29, and LAMP1 were greatly decreased after treatment with proteinase. On the other hand, in the case of CD63, it was confirmed that degradation by treatment with proteinase was not effective. As known in the related art, it seemed that a proteinase recognition site of CD63 was excessively glycosylated or CD63 had an "inside-out" topology, which was not affected by proteinase.

Since all protein markers other than CD63 decreased in expression levels on the surfaces of CDVs and exosomes according to proteinase treatment, the cellular uptake efficiency was compared to determine whether the cellular uptake rate was changed according to such a decrease. CDVs and exosomes treated with proteinase were fluorescent-labeled in the same manner as in Example 2, and cellular uptake rates were confirmed in BT549, a human triple-negative breast cancer cell line.

As shown in FIG. 6, it was confirmed that the cellular uptake rates were significantly decreased in all experimental groups treated with proteinase K, as protease. In particular, CDVs showed an uptake rate similar to or lower than that of cell-secreted exosomes according to proteinase treatment, which showed a decrease in uptake rate of about 50% or more. HEK-CDVs showed a much larger decrease in uptake rate than UCMSC-CDVs. On the other hand, a relatively small decrease in uptake rate was observed in exosomes (UCMSC-Exo; HEK-Exo) after surface proteolysis. These results showed that CDV membrane surface protein markers were involved in cellular uptake of CDVs, regardless of a cell source.

### Example 5. Comparison of cellular uptake rate according to membrane surface protein blocking

Surface proteins of CDVs and exosomes were blocked using antibodies of selected markers based on known exosome surface protein markers, and then cellular uptake rates were compared. Among the surface protein markers of exosomes, the experiment was first performed mainly with markers well known in previous studies, and among organelle markers, a lysosomal protein LAMP1 was used. CDVs and exosomes derived from UCMSC and HEK293 (suspension) cells were used as samples.

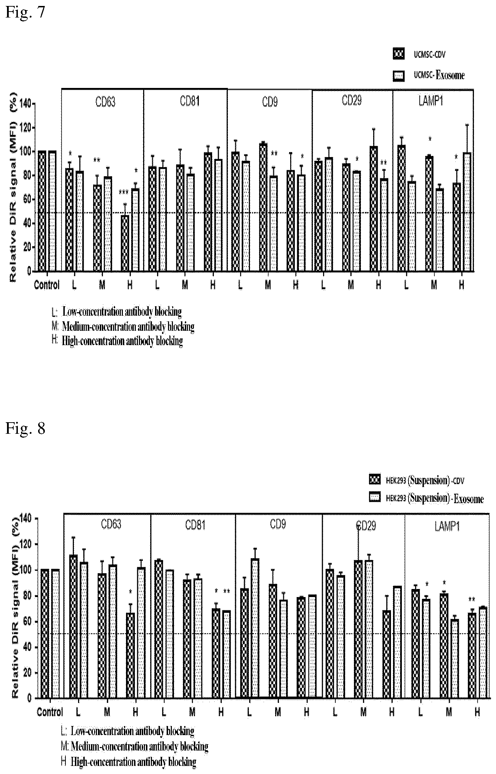

For each sample, 2 × 10⁹ particles of CDVs and exosomes (hereinafter, DiR-CDVs; DiR-exosomes) prepared from DiR-labeled UCMSC and HEK293 (suspension) cells according to the method of Example 2 reacted with antibodies (antibodies against CD63, CD29, CD81, CD9, CD29 and LAMP1, BD Biosciences, MA, US) at concentrations of 1 : 100; 1 : 1,000; and 1 : 10,000 for 30 minutes at 4°C under dark conditions, respectively. Thereafter, each of the DiR-CDVs and DiR-exosomes treated with the antibody at each concentration was immediately used in an experiment for measuring cellular uptake rates. 2 × 10⁴ of BT549 cells as a human triple-negative breast cancer cell line were spread on a 48-well plate and cultured for 17 hours or more to be adhered. Triple negative breast cancer cells were separately treated with DiR-CDVs and DiR-exosomes blocked with each antibody at 1 × 10⁵ particles/cell and cultured at 37°C for 24 hours. Fluorescence intensities were measured using a flow cytometer to quantify the cellular uptake rates of control groups (antibody-untreated DiR-CDVs, antibody-untreated DiR-exosomes), antibody-treated DiR-CDVs, and antibody-treated DiR-exosomes, and then graphed. Since CDVs and exosomes had different levels of cellular uptake, for comparative analysis, the values of each control group (antibody-untreated DiR-CDVs, antibody-untreated DiR-exosomes) were quantified to 100%, and then DiR expression was relatively compared. The results of confirming the cellular uptake rates by blocking surface protein markers of UCMSC-CDV and UCMSC-exosomes were shown in FIG. 7, and the results of confirming the cellular uptake rates by blocking HEK293 (suspension)-CDV and HEK293 (suspension)-exosome surface protein markers were shown in FIG. 8.

As shown in FIG. 7, in the case of CD63 and LAMP1, the degree of cellular uptake of UCMSC-CDVs gradually decreased as the antibody concentration gradually increased. In particular, in the case of CD63, when CD63 was blocked with an anti-CD63 antibody, the cellular uptake efficiency of UCMSC-CDVs decreased most noticeably to 50% or more. On the other hand, in the case of CD81, CD9, and CD29, the cellular uptake rate of UCMSC-CDVs did not show a large change according to each antibody concentration. In addition, in the case of UCMSC-exosomes, when specific surface protein markers CD63, CD9, and CD29 were blocked with each antibody, the cellular uptake rate decreased by approximately 20 to 30% in a concentration-dependent manner, but the decrease rate was not greater than that when UCMSC-CDVs were blocked with a CD63 antibody.

As shown in FIG. 8, it was confirmed that the cellular uptake rate of HEK293 (suspension)-CDVs according to blocking of the CD63 antibody decreased in a concentration-dependent manner of the antibody, and when treated with a high concentration of the antibody, the cellular uptake was decreased to about 40%. In addition, unlike UCMSCs, it was observed that the cellular uptake of HEK293 (suspension)-CDVs and HEK293 (suspension)-exosomes was inhibited in an antibody concentration-dependent manner by the blocking of CD81. In addition, the cellular uptake of HEK293 (suspension)-CDV according to the blocking of the LAMP1 antibody was decreased in a concentration-dependent manner, like the case of UCMSC-CDVs.

Through the results, it was confirmed that the characteristics of CDVs exhibiting high cellular uptake capacity as compared to cell-secreted exosomes were based on the expression of membrane surface protein markers different from those of exosomes. It is considered that a cellular uptake mechanism of CDVs or cell-secreted exosomes varies depending on a type of cell, and CDVs and cell-secreted exosomes have different markers involved in cell uptake, respectively. On the other hand, it was confirmed that CD63 and LAMP1 affected the cellular uptake capacity of CDVs regardless of a cell type.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A method for producing cell-derived vesicles (CDVs) with increased cellular uptake capacity comprising producing the cell-derived vesicles by migrating a sample containing cells into micropores.

2. The method of claim 1, wherein the cell-derived vesicles are increased in expression of at least one membrane surface protein marker selected from the group consisting of CD29, flotillin-1, CD63, LAMP1, Calnexin and GM130; or decreased in expression of at least one membrane surface protein marker selected from the group consisting of CD81 and CD9 as compared with cell-secreted exosomes.

3. The method of claim 1, wherein the micropores are migrated sequentially from a large pore size to a small pore size.

4. The method of claim 1, wherein the micropores are migrated by a pressure.

5. The method of claim 1, wherein the cell-derived vesicles are nucleated cell-derived vesicles.

6. The method of claim 1, wherein the cell-derived vesicles are stem cells, undifferentiated cells, immune cells, somatic cells, induced pluripotent stem cells, or germ cell-derived vesicles.

7. The method of claim 1, wherein the cell-derived vesicles are one or more cell-derived vesicles selected from the group consisting of umbilical cord-derived mesenchymal stem cells, Wharton's jelly-derived mesenchymal stem cells, placenta-derived mesenchymal stem cells, tonsil-derived mesenchymal stem cells, adipose-derived mesenchymal stem cells, cord blood-derived mesenchymal stem cells, bone marrow-derived mesenchymal stem cells, and embryonic kidney cells.

8. Cell-derived vesicles with increased cellular uptake capacity **characterized by** increasing expression of at least one membrane surface protein marker selected from the group consisting of CD63 and LAMP1 as compared to cell-secreted exosomes.

9. The cell-derived vesicles with increased cellular uptake capacity of claim 8, wherein the cell-derived vesicles are **characterized by** increasing in expression of at least one membrane surface protein marker selected from the group consisting of CD29, flotillin-1, Calnexin and GM130 as compared to cell-secreted exosomes.

10. The cell-derived vesicles with increased cellular uptake capacity of claim 8, wherein the cell-derived vesicles are decreased in expression of at least one membrane surface protein marker selected from the group consisting of CD81 and CD9 as compared to cell-secreted exosomes.

11. The cell-derived vesicles with increased cellular uptake capacity of claim 8, wherein the cell-derived vesicles are produced by a method including producing the cell-derived vesicles by migrating a sample containing cells into micropores.

12. Cell-derived vesicles with increased cellular uptake capacity produced by a method comprising producing the cell-derived vesicles by migrating a sample containing cells into micropores.

13. A composition for use in delivering an active ingredient comprising the cell-derived vesicles with increased cellular uptake capacity of any one of claims 8 to 12.

14. A method for delivering an active ingredient into a subject *in vitro* or *ex vivo* comprising treating the cell-derived vesicles with increased cellular uptake capacity of any one of claims 8 to 12 comprising the active ingredient to a subject.

15. A method for delivering an active ingredient into a subject comprising treating the cell-derived vesicles with increased cellular uptake capacity of any one of claims 8 to 12 comprising the active ingredient to a subject.
